# EUROPEAN PATENT SPECIFICATION

(11) **EP 3 386 581 B1**
(45) Date of publication and mention of the grant of the patent: **24.04.2024**
(21) Application number: 16873766.6
(22) Date of filing: 07.12.2016
(51) Int. Cl.: A61M 35/00

(54) **APPARATUS AND METHODS FOR CONTROLLING TISSUE OXYGENATION FOR WOUND HEALING AND PROMOTING TISSUE VIABILITY**
VORRICHTUNG UND VERFAHREN ZUR STEUERUNG DER SAUERSTOFFANREICHERUNG IN EINEM GEWEBE ZUR WUNDHEILUNG UND ZUR FÖRDERUNG DER GEWEBE-LEBENSFÄHIGKEIT
APPAREIL ET MÉTHODES DE RÉGULATION DE L'OXYGÉNATION DES TISSUS POUR LA CICATRISATION DES PLAIES ET L'AMÉLIORATION DE LA VIABILITÉ DES TISSUS

(30) Priority: 07.12.2015 US 201514961587
(43) Date of publication of application: 17.10.2018
(73) Proprietor: Electrochemical Oxygen Concepts, Inc., San Antonio, TX 78249 (US)
(72) Inventor: WELLS, Michael C., San Antonio, Texas 78257 (US); PARKER, Mark, Houston, Texas 77041 (US); CLARIUS, Daniel J., Missouri City, Texas 77459 (US); PARKER, Andrew, Missouri City, Texas 77459 (US); PUNDOLE, Faraidoon, Sugar Land, Texas 77479 (US); WOODS, Tom, Friendswood, Texas 77549 (US); NIEDERAUER, Mark, San Antonio, Texas 78261 (US); DALEY, James P., San Antonio, Texas 78247 (US)
(74) Representative: Carpmaels & Ransford LLP
(86) International application number: PCT/US2016/065378
(87) International publication number: WO 2017/100320

(56) References cited:
- US-A1- 2008 003 299
- US-A1- 2008 308 100
- US-A1- 2009 112 170
- US-A1- 2013 144 227
- US-B2- 7 322 971

## Description

### BACKGROUND

### Field

Embodiments disclosed herein relate to tissue treatment systems, specifically to tissue oxygenation systems for accelerating the healing of damaged tissue and promoting tissue viability. The tissue oxygenation system described herein may be used to treat skin ulcerations due to diabetes, venous stasis, post surgical infections, gangrenous lesions, pressure ulcers, infected residual limbs, skin grafts, burns, frostbite, and/or a variety of other wounds and bodily conditions known in the art.

When tissue is damaged, a wound results and a healing process begins. The term "wound" may include, but is not limited to, chronic wounds, traumatic wounds, surgically created wounds, and/or other wounds known in the art. Optimal metabolic function of cells to repopulate tissue in the wound requires that oxygen be available for all phases of wound healing, and the more layers of tissue that are damaged, the greater the risk for complications to occur in the wound healing process.

Difficult-to-heal wounds encounter barriers to the wound healing process and typically experience delays in one or more of the phases of wound healing. One of the most common contributing factors to venous leg ulcers, diabetic foot ulcers, and pressure ulcers experiencing delays in the healing process is the problem of chronic wound ischemia. Chronic wound ischemia is a pathological condition that restricts blood supply, oxygen delivery, and blood request for adequate oxygenation of tissue, operating to inhibit normal wound healing.

In practice the standard of care for treating difficult-to-heal wounds typically involves the use of an advanced wound dressing or combinations of advanced wound dressings that provide a dressing treatment system. An advanced dressing is positioned on the wound site and/or around the surrounding intact skin to provide a wound site enclosure. An advanced wound dressing typically includes materials having properties for promoting moist wound healing, managing wound exudate, and helping control wound bioburden. The typical material components in combination further include properties for providing limited moisture vapor permeability. The lower the dressing's moisture vapor permeability, or the more occlusive the dressing, the lower the amount of ambient air and respective oxygen that will be available to the wound bed. 100% oxygen exerts a partial pressure of 760 mm Hg. Ambient air is comprised of about 21 % oxygen, thereby exerting a partial pressure of oxygen at about 159 mm Hg. A typical advanced wound dressing or wound dressing system including lower moisture vapor permeable materials impacts the available oxygen for the wound site, thereby limiting the partial pressure of oxygen at the enclosed wound site at about 10-60 mm Hg. As such, fresh air is provided to the wound site only when the dressing is changed, and a dressing may remain covering the wound site for up to seven days before a dressing change is required. The moisture vapor permeability property of an advanced wound dressing providing a reduced oxygen wound environment thereby works against the optimal metabolic function of cells to repopulate tissue in the wound, which would benefit from oxygen being available during all phases of wound healing.

Prior art methods of tissue oxygenation for difficult-to-heal wounds typically include topical hyperbaric oxygen applied intermittently or continuously. Intermittent topical hyperbaric oxygen is a method of tissue oxygenation that includes a sealed extremity or partial body chamber and a connected source of high flow, pure oxygen. The affected limb or affected body area is positioned in the sealed extremity chamber or partial body chamber so that the oxygen source supplying the chamber may provide the patient topically up to 100% oxygen at flow rates that may exceed 300 liters per hour to pressurize the interior of the chamber up to 1.05% normal atmospheric pressure, thereby increasing the available oxygen for cellular processing at the affected wound site. During the oxygen application, the partial pressure of oxygen exerted inside the topical or partial body chamber may attain 798 mm Hg, and the topical hyperbaric oxygen may be applied for about 90 minutes. Prior art also teaches a plurality of methods to apply topically hyperbaric oxygen intermittently. A partial body chamber for treating sacral wounds has been described in U.S. Pat. No. 4,328,799 to LoPiano (1980), whereby oxygen is applied from a stationary supply tank into the interior of the chamber through connected tubing. A similar method of applying topical hyperbaric oxygen is described in U.S. Pat. No. 5,478,310 to Dyson-Cantwell (1995), whereby oxygen is applied from a stationary supply tank into the interior of the disposable extremity chamber through connected tubing. These and similar methods of applying intermittent topical hyperbaric oxygen are restrictive, cumbersome, can only supply oxygen to the affected area intermittently with no systemic application, and can only be applied with a minimal increase in atmospheric pressure (about 5%). Therefore the effect of the oxygen therapy on the wound tends to be minimal, which is evidenced by the lack of commercial success from topical hyperbaric oxygen extremity chambers.

Both U.S. Pat. No. 5,578,022 to Scherson (1996) and U.S. Pat. No. 5,788,682 to Maget (1998) describe disposable devices utilizing transmission of gases in ionic form through ion specific membranes to apply supplemental oxygen directly to the wound bed. These devices are described as battery powered, disposable, oxygen producing bandages and methods that are applied directly over the wound. Both devices include electrochemical oxygen generation using variations of the same 4 electron formula originally developed for NASA in U.S. Pat. No. 3,489,670 to Maget (1970). The amount of oxygen that can be applied to the wound is typically 3 milliliters per hour. Specific oxygen flow rates are generated by means of corresponding specific, preselected battery sizes and specific, prescribed amperages, and describes these disposable devices as either "on or off." The prior art also describes disposable devices without means to sense temperature changes in the wound site oxygen environment, and does not provide a means to deliver a varying or adjustable oxygen flow rate without requiring the patient to obtain and apply a new device with a new battery having a specific amperage. Additional limitations are also associated with the use of a fixed non-variable oxygen flow rate. >Insert Page 4a and 4b

US2009112170A1 describes a non-invasive tissue oxygenation system for accelerating the healing of damaged tissue and to promote tissue viability. The system is comprised of a lightweight portable electrochemical oxygen concentrator, a power management system, microprocessors, memory, a pressure sensing system, a temperature monitoring system, oxygen flow rate monitoring and control system, a display screen and key pad navigation controls as a means of providing continuous variably controlled low dosages of oxygen to a wound site and monitoring the healing process. A kink resistant oxygen delivery tubing, whereby the proximal end is removably connected to the device and the distal end with holes or a flexible, flat, oxygen-permeable tape is positioned on the wound bed as a means of applying oxygen to the wound site. The distal end of the tube is in communication with the electrochemical oxygen concentrator and wound monitoring system to communicate temperature and oxygen partial pressure information. A moisture absorbent dressing is positioned over the distal end of the tubing on the wound site and a reduced moisture vapor permeable dressing system is positioned whereby covering the moisture absorbent dressing, distal end of tubing and wound site creating a restricted airflow enclosure. The restricted airflow enclosure allows the out-of-the-way control and display unit to provide a controlled hyperoxia and hypoxia wound site for accelerated wound healing.

US2008003299A1 describes a method of treatment of a wound, said method comprising the step of injecting an effective amount of an oxygen-containing fluid into said wound at a location below a surface of said wound, said step of injecting being carried out through a conduit having one or more outlets for said fluid, wherein substantially all of said outlets are positioned below the surface of the wound during said step of injecting.

US2008308100A1 describes a patient treatment system and method comprising a patient circuit, a pressurizing flow module disposed in the patient circuit, a humidifier disposed in the patient circuit, a monitor, sensors within the patient circuit, and a processor. The patient circuit delivers a flow of gas from a gas source to an airway of a patient. The pressurizing flow module elevates the pressure of the gas within the patient circuit. The humidifier elevates the humidity of the gas in the patient circuit to a circuit humidity level. The monitor monitors at least one parameter of the gas from the gas source. The sensors generate corresponding signals that can be processed to estimate a rate at which vapor is added to the gas in the patient circuit. The processor determines the humidity level of the gas in the patient circuit downstream from the humidifier based on the signals generated by the sensors and the at least one parameter of the gas in the gas source.

US7322971 is directed to devices and methods of using a surgical drain, and more particularly to a surgical drain having at least one sensor for monitoring and/or recording the condition of the anatomical site or fluid emitted from the site where the surgical drain is placed.

US2013144227 describes systems and methods for treating a plurality of tissue sites using reduced-pressure which include a multi-port therapy unit. The system includes a reduced-pressure source fluidly coupled to a dressing at each tissue site through the multi-port therapy unit.

No prior art tissue oxygenation device provides continuous oxygen adjustability to a patient's wound(s) to create a controlled hyperoxia and hypoxia wound environment for damaged tissue to accelerate wound healing and promote tissue viability. Specifically, nothing in the prior art teaches continuous oxygen adjustability based on actual flow rate, partial pressures at the wound site, and, where beneficial, temperatures, pH values, and/or other local conditions at the wound site.

### SUMMARY AND DESCRIPTION

Embodiments disclosed herein include improved tissue oxygenation devices and wound monitoring systems. Those embodiments generally comprise an oxygen delivery, micro-bore tube for placement at or near the wound bed, and a wound dressing covering the tubing and wound site to provide a restricted air flow enclosure. The tubing may have multiple holes at or near the distal end of the tubing, and may include a generally flat, flexible, oxygen-permeable tape or membrane section attached at the distal end of the tubing. The tubing may be flexible with a kink resistant inner lumen. The tubing may include a temperature sensor. The tubing may include a pressure sensor. The tubing may include a partial pressure of oxygen sensor. The proximal end of the tubing is connected to a source of oxygen. The proximal end of the tubing may have a port Leur-type locking mechanism for an airtight seal during application of the oxygen and for removal from the oxygen source during application of a dressing. A source of oxygen is in communication with the proximal and distal ends of the tube, and may be provided by an electrochemical oxygen concentrator supplied by alternating or direct current, and a power management device using a power management protocol. The variable electrochemical oxygen concentrator is used in accordance with the embodiments of the present disclosure by varying the oxygen flow rate to meet varying target parameters at the wound site. The oxygen flow rate is adjusted by a system that periodically or continuously monitors the wound bed pressure and, where beneficial, the temperature environment. Additionally, the system may monitor the tubing pressure and adjust the oxygen flow rate in accordance to target set points. Adjusting oxygen flow in response to monitored changes in wound site oxygen and target oxygen pressure and, where beneficial, wound site temperature, humidity, pH or perfusion, provides a controlled hyperoxia wound environment which may shorten the healing process. The present monitoring system may further provide an alarm when the pressure, temperature, humidity, pH, perfusion, or flow rates have gone out of range.

In some situations, excessive oxygen pressure (i.e., greater than 22 mm of Hg) can occlude arterial circulation and lead to decreased local tissue circulation and, in some cases, local tissue damage. Embodiments of the device design may address how the device controls oxygen pressure such that pressures do not exceed a safe limit. Unlike a conventional topical oxygen chambers for extremities, the present system does not create a sealed, oxygen rich environment inside a chamber in which a patient's limb is inserted. Instead, oxygen may be generated by a Proton Exchange Membrane within the portable device and delivered to the wound site via micro-bore tubing at a rate of 3 ml/hr to 100 ml/hr. Because the wound site is covered only by occlusive wound dressing instead of the entire limb being inserted into a sealed chamber, such oxygen pressure at the site and around the limb may never exceed normal atmospheric pressure.

In some embodiments, the device may have a backlight display terminal or touch screen liquid crystal display, a data input key pad or device function control buttons, a wound or housing temperature monitoring system, a wound or device humidity monitoring system, a battery and oxygen pressure alarm system, a digital camera, a patient data input and memory system, a data port, wireless data access, and/or the other components discussed below.

### BRIEF DESCRIPTION OF THE DRAWINGS

A more complete understanding of the present disclosure and advantages thereof may be acquired by referring to the following description taken in conjunction with the accompanying figures, wherein:
FIG. 1 is a perspective view illustrating an embodiment of a tissue oxygenation system.
FIG. 1A is a front view illustrating an embodiment of the tissue oxygenation system of FIGS. 1 and 1A being used to treat multiple wounds on a patient.
FIG. 1b is a cross section view illustrating an embodiment of a wound site showing a distal end of oxygen delivery tubing that may be used in the tissue oxygenation system of FIG. 1.
FIG. 2 is a side perspective view illustrating an embodiment of the distal end of the oxygen delivery tubing of Fig. 1A showing a generally flat, flexible, oxygen-permeable tape or membrane section affixed to the oxygen delivery tubing.
FIG. 2A is an end view illustrating an embodiment of the oxygen delivery tubing of Fig. 1A.
FIG. 3 is side elevation view illustrating an embodiment of the distal end of the oxygen delivery tubing of FIG. 2A.
FIG. 4 is a perspective view illustrating an embodiment of a handset that may be used in the tissue oxygenation system of FIG. 1.
FIG. 5 is a schematic view illustrating an embodiment of the tissue oxygenation system of FIG. 1.
FIG. 6 is a top plan view illustrating an embodiment of the electrolyzer/concentrator that may be used in the tissue oxygenation system of FIG. 1.
FIG. 6A is a side elevation plan view illustrating an embodiment of the electrolyzer/concentrator of FIG. 6.
FIG. 6B is an exploded perspective view illustrating an embodiment of the electrolyzer/concentrator of FIG. 6.
FIG. 7 is a flow chart illustrating an embodiment of a method for controlling tissue oxygenation that may be used with the tissue oxygenation system of FIG. 1.

### DETAILED DESCRIPTION

Several embodiments of tissue oxygenation systems for promoting the healing of damaged tissue and tissue viability will now be described in detail with reference to the figures.

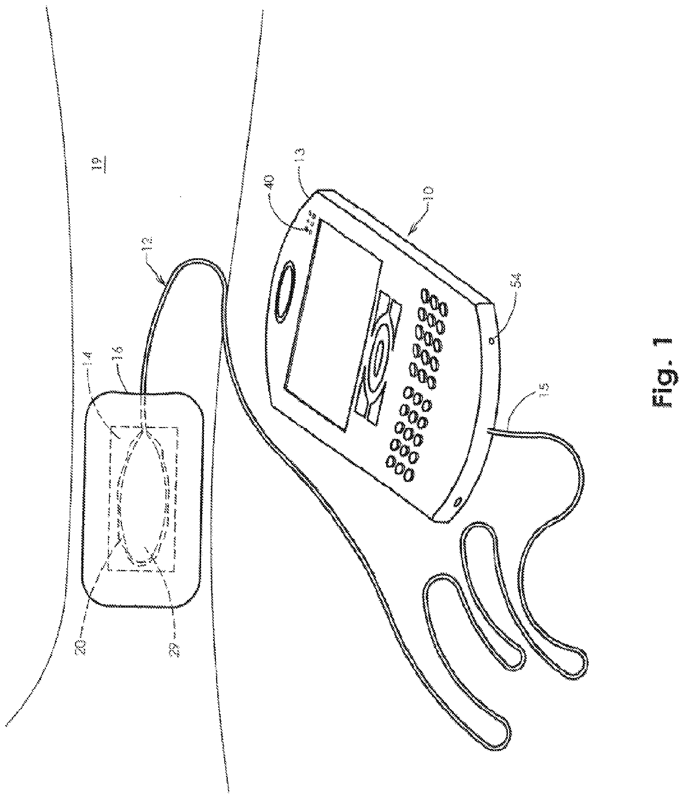

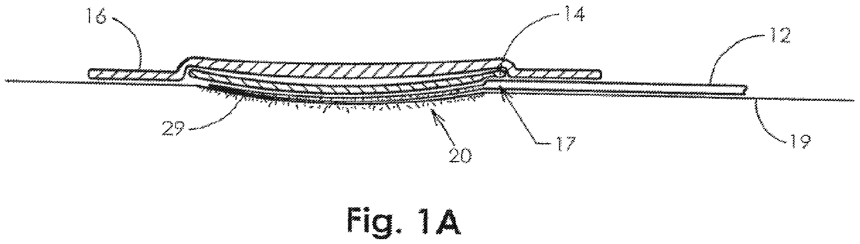

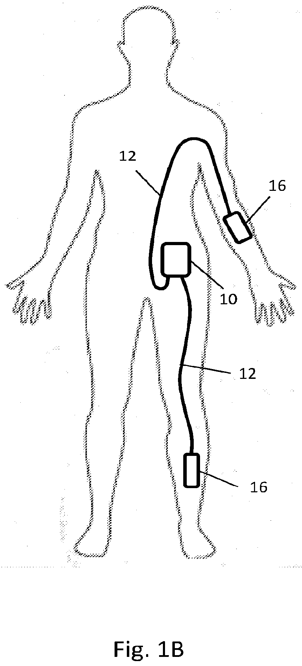

FIG. 1 is a perspective view of several components of the tissue oxygenation system of the present disclosure providing oxygen to a wound. The illustrated embodiment includes a monitoring unit 10, an electrochemical oxygen concentrator 11, oxygen delivery tubing 12, a moisture absorbent dressing 14, and vapor permeable dressing 16. The oxygen delivery tubing 12 may be connected (at the proximal end 15 of the relatively long, kink-resistant tubing illustrated in Fig. 1) to the monitoring unit 10. In an embodiment, the monitoring unit 10 is provided in a relatively small, lightweight housing that is portable and may be discretely worn by a patient in a pocket, attached to a belt, and/or otherwise carried by the patient in various manners known in the art.

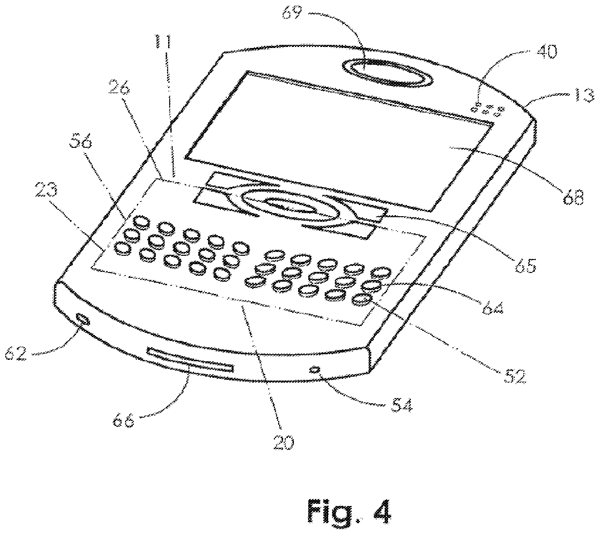

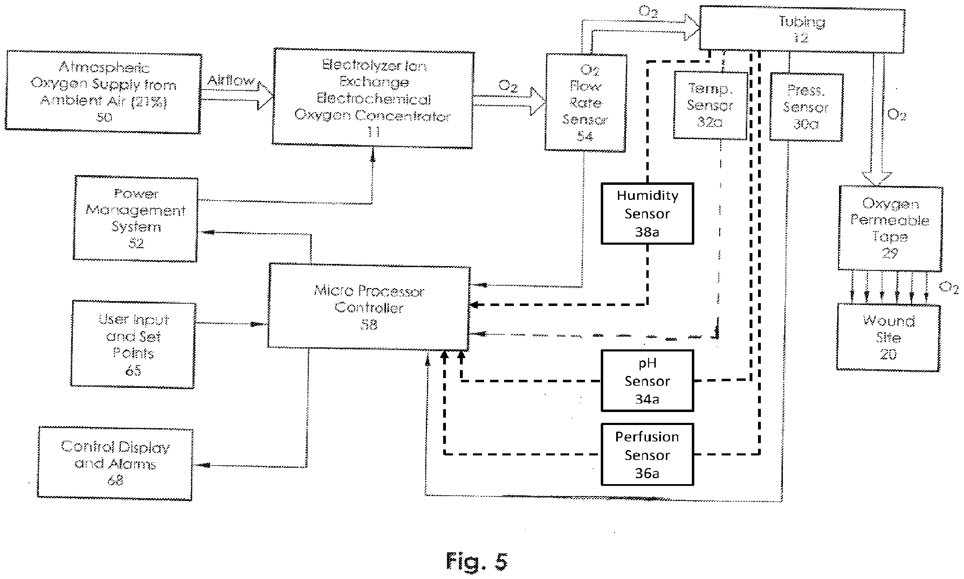

The monitoring unit 10 may include, within the housing 13, any or all of a microprocessor 58 (see FIGS. 4 and 5), a power management system 52, one or more pressure sensors 30a or pressure sensor interfaces, one or more temperature sensors 32a or temperature sensors interfaces, one or more flow rate sensors 54 or flow rate sensor interfaces, an input port 62, and a user entry port 66. The monitoring unit 10 may further include, within the housing 13, any or all of one or more pH sensors 34a or pH sensor interfaces, one or more perfusion sensors 36a or perfusion sensor interfaces, and one or more humidity sensors 38a or humidity sensor interfaces. The electrochemical oxygen concentrator 11 may be disposed within the housing 13. The microprocessor 58 may function to control power, collect various readings from flow rate sensor(s) 54, pressure sensor(s) 30a, pH sensor(s) 34a, perfusion sensor(s) 36a, humidity sensor(s) 38a, and temperature sensor(s) 32a, either included in the housing 13 or coupled to the corresponding interfaces in the housing 13, control ionic purification of ambient air by the electrochemical oxygen concentrator 11 for delivery to the tubing 12, and provide information on the informational display of the monitoring unit 10. The microprocessor 58 is capable of receiving data through the user entry port 66 and the input port 62, wirelessly via BLUETOOTH^{®}, WiFi^{®}, and/or other wireless technologies known in the art, and/or using other communication system, with that data including information related to specific patients, reprogramming information if there is a system malfunction with the device, and/or any other information that would be apparent to one of skill in the art in possession of the present disclosure.

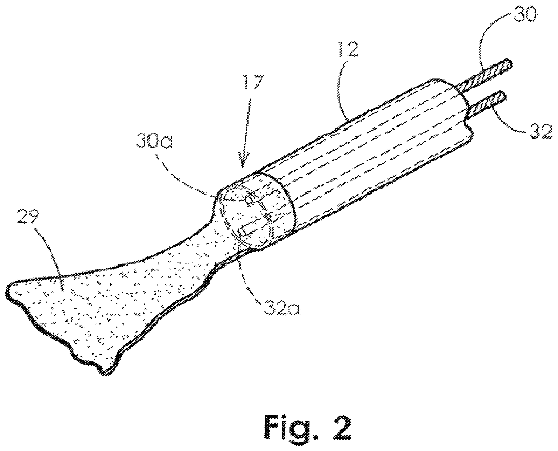

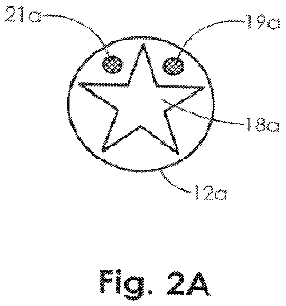

As may be further seen in FIGS. 1A and 2, a distal end 17 of the tubing 12 may include or be coupled to a soft, flexible, oxygen permeable tape or membrane section 29 that may be placed on or near damaged tissue or wound site 20 on a patient's limb 19, and covered with a moisture absorbent dressing 14, which is further covered by a reduced vapor pressure, permeable, occlusive dressing 16.

In an embodiment, oxygen may be delivered to the wound site 20 through kink-resistant tubing 12 that is connected at its proximal end 15 to the outlet of the oxygen concentrator in the housing 13 of the monitoring unit 10. Furthermore, in some embodiments, the oxygen concentrator in the housing 13 of the monitoring unit 10 may include a plurality of oxygen outlets, and a plurality of the kink-resistant tubing 12 may be connected at their proximal ends 15 to respective outlets of the oxygen concentrator, with their respective distal ends 17 placed on or near a plurality of different damaged tissue or wound sites 20 on a patient and covered by a reduced vapor pressure, permeable, occlusive dressing 16, as illustrated in Fig. 1B. While only two wounds sites on the patient are illustrated as being treated in Fig. 1B, one of skill in the art in possession of the present disclosure will recognize that any number of wounds may be treated in a similar manner for example, by increasing the oxygen output of the oxygen concentrator, providing more oxygen concentrators in the housing 13 of the monitoring unit 10, etc.

As illustrated in Fig. 2, in an embodiment, on the distal end 17 of each tubing 12, a soft, flexible oxygen-permeable flat tape or membrane 29 may be connected. Sensor wires 30 and 32 may extend through the lumen of each tubing 12 to communicate with any of the sensors or sensor interfaces included in the housing 13 of the monitor unit 10 discussed above. For example, the sensor wires 30 and 32 may communicate with temperature sensor(s) 32a, oxygen partial pressure sensor(s) 30a, flow rate sensor(s) 54, humidity sensor(s) 38a, pH sensor(s) 34a, perfusion sensor(s) 36a, and/or other sensors disposed at each wound site and provide that information to the microprocessor 58. In other embodiments, the sensor wires may be eliminated and temperature, pressure, flow rate, pH, perfusion, humidity, and/or other wound environment measurements may be measured within the monitor unit housing at each outlet of the oxygen concentrator.

As illustrated in fig. 2A, in some embodiments tubing 12a may include several lumens, sensors, and/or other subsystems as may be required to effectively monitor wound treatment. Specifically, FIG. 2A illustrates an end view of the tubing 12a, and depicts a tubing 12A with a length capable of connecting to the output side of any of the outlets of the electrochemical oxygen concentrator 11 housed within the monitoring unit 10. Such tubing lengths allow the monitoring unit to be worn discreetly while oxygen is continuously delivered to the wound site 20. An inner lumen or bore 18a of the tubing may be provided with the star-like configuration of Fig. 2A to prevent kinking of the tubing and/or enable oxygen flow when the tubing 12a is bent, although other configurations of the lumen 18a may provide similar benefits. An oxygen partial pressure sensor 19a that may be positioned at or near the wound site may be disposed within the tubing 12A and be in communication with a pressure monitoring system and/or microcontroller to allow for oxygen flow rate adjustment, visual pressure display, out-of-range alarms, and/or other benefits that would be apparent to one of skill in the art. A temperature sensor 21a that may also be positioned at or near the wound site may be disposed within the tubing 12a and be in communication with a temperature monitoring system and/or microcontroller to allow for visual display of temperature, an out-of-range alarm, to allow for oxygen adjustment via the microprocessor 58 as is appropriate, and/or to provide other benefits that would be apparent to one of skill in the art. Furthermore, the pH sensor, perfusion sensor, and/or humidity sensor that may also be positioned at or near the wound site may be similarly disposed within the tubing 12a and in communication with a pH monitoring system, perfusion monitoring system, humidity monitoring system, and/or microcontroller to allow for visual display of pH, perfusion, relative humidity, out-of-range alarms, to allow for oxygen adjustment via the microprocessor 58 as is appropriate, and/or to provide other benefits that would be apparent to one of skill in the art.

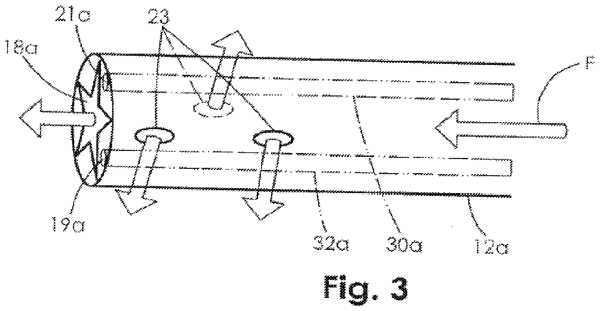

FIG. 3 is a side view of an embodiment of the distal end of the tubing 12a, which may include a plurality of holes 23 formed along the side of the distal end of the tubing 12a to aid in the delivery of oxygen to the wound. In use, the oxygen may be provided in a flow F through the tubing 12a to the wound site and may enter the wound bed through the multiple holes 23. The oxygen may also flow through the distal end of star shaped lumen 18a, but in some embodiments the multiple holes at the distal end of the tubing 12a may allow for improved flow of oxygen to the wound site 20.

FIG. 1A illustrates a wound site 20, with the distal end 17 of the oxygen delivery tubing 12 having the oxygen distribution tape 29 placed over or near the wound site 20, although in some embodiments the tape 29 may be placed centrally on the wound site for optimal delivery of oxygen to the damaged tissue. In some embodiments, the wound may be saturated with nearly 100% O2. A moisture absorbent dressing 14 may be placed at the wound site to cover the tape end of the oxygen delivery tubing 12 and wound site. One skilled in the art in possession of the present disclosure will appreciate that a variety of moisture absorbent dressings that provide the typical standard of care protocol for a difficult-to-heal wound may be utilized with the oxygen delivery tubing 12 as discussed above. A reduced moisture vapor permeable dressing 16 may then be used to cover the moisture absorbent dressing 14, the tape end of the tubing 12, and the wound site 20 in order to create a restricted airflow enclosure. In some embodiments, the reduced moisture vapor permeable dressing 16 may be transparent and may be considered an occlusive dressing. The occlusive dressing may operate to trap the oxygen over the wound site to create and maintain oxygen rich environment. In some embodiments, the local partial pressure of oxygen at the wound site 20 may be increased from a low range of 10 to 60 mm Hg to an oxygen rich environment range of 200 to 760 mm HG, and that increased available oxygen may be metabolized at the cellular level to stimulate an increase in growth factors, epithelialization, granulation tissue, glycosaminoglycan production, collagen synthesis, and/or other wound healing factors known in the art. In an embodiment, the oxygen partial pressure at the wound site may be communicated to the pressure monitoring system and/or microprocessor in the housing 13. The oxygen partial pressure sensor may supply data to the microprocessor 58 that the microprocessor 58 may use to control the power flow (amperage) to the concentrator 11, which may in turn cause the concentrator to increase or decrease its production of O2 to change the O2 flow rate to the wound site. In some embodiments, the oxygen partial pressure sensor may be conductivity sensor, but other systems for measuring this pressure may be utilized while remaining within the scope of the present disclosure.

FIG. 4 is a perspective view of a handset that may house the components of the tissue oxygenation system of the present disclosure, while FIG. 5 is a schematic view of those components. In use, the monitor housing 13 may draw ambient air 50 with about 21% oxygen through the air inlet 40 by means of an electrochemical process. The ambient air 50 may then pass through an ion exchange oxygen concentrator 11, which operates to concentrate the oxygen in the ambient air 50 to create an airflow that is 99% pure oxygen. The power management system 52 may control the electrical current supplied to the ion exchange oxygen concentrator 11, thereby making the oxygen flow rate conform to the amount of current supplied to the ion exchange oxygen concentrator 11, i.e., increasing electrical current will increase the electrochemical process and thereby increases the respective oxygen flow rate to the wound site 20, and decreasing the electrical current will decrease the electrochemical process thereby decreasing the respective oxygen flow rate to the wound site. In some embodiments, the power management system 52 may include lithium batteries (for example, 3.7 v) and a regulator that operates to vary the current over a range of approximately 20 milliamps to approximately 140 milliamps, while in other embodiments, that range may be approximately 7 milliamps to approximately 70 milliamps. This range of current variation may result in O2 flow rates in the range between approximately 3.0 milliliters/hour to approximately 20.0 milliliters/hour in some embodiments, while in other embodiments that range may be between approximately 3.0 milliliters/hour to approximately 10.0 milliliters/hour.

The concentrated O2 then exits the housing through the oxygen delivery port 54. The proximal end 15 of the oxygen delivery tubing 12 is connected with an oxygen delivery port 54 with Leur-type locking fitting, or other fitting known in the art, to maintain an airtight seal with the tubing 12 and allows the tubing 12 to deliver that concentrated O2 to the wound site.

As illustrated in FIGS. 2 and 5, the pressure sensor 30a or 19a and, in some embodiments, the temperature sensor 32a/21a in the tubing 12 or 12a are in communication with the microprocessor 58. In addition, a pH sensor 34a, a perfusion sensor 36a, and/or a humidity sensor 38a may also be in communication with the microprocessor 58. The microprocessor 58 may communicate with the power management system 52 to adjust the oxygen flow rate (sensed by sensor 54) to the wound site per programmed algorithms to optimally meet changes in the patients oxygen wound healing requirements. In one embodiment, if a plurality of tubing 12 or 12a is connected between the oxygen concentrator and multiple wound sites (as illustrated in Fig. 1B, the microprocessor 58 may communicate with the power management system 52 to adjust the oxygen flow rates (sensed by respective sensors 54) to each individual wound site per programmed algorithms to optimally meet changes in the patient's oxygen wound healing requirements for each wound, which may be different and thus may require different oxygen flow rates and/or other treatment characteristics that would be apparent to one of skill in the art in possession of the present disclosure.

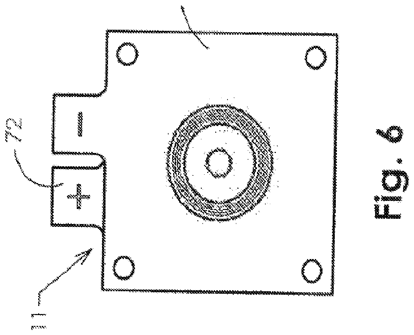

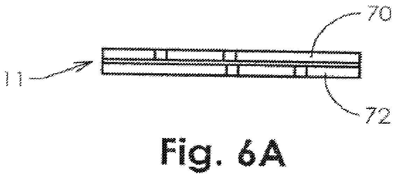

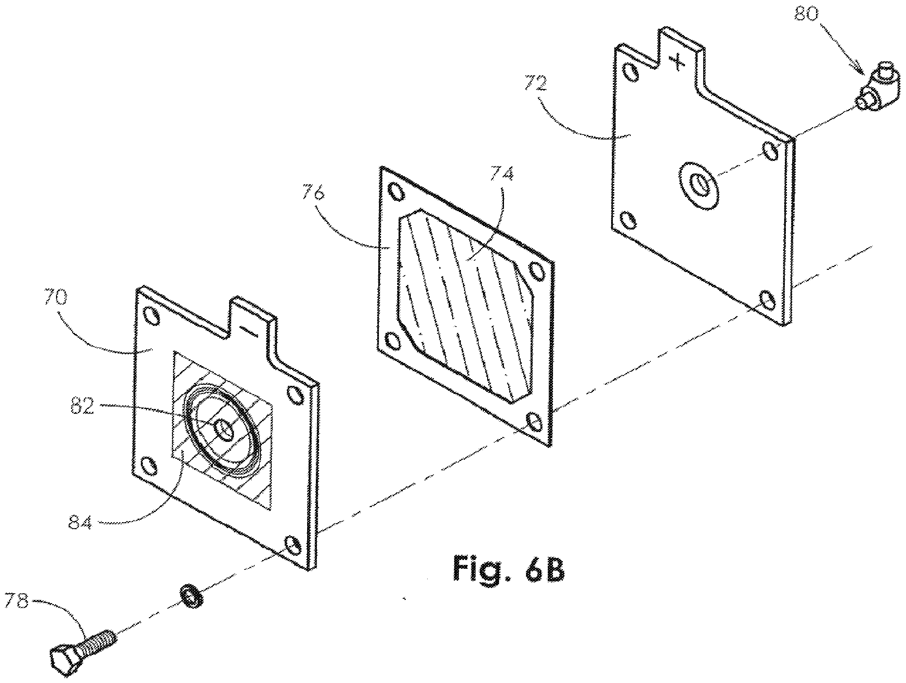

Turning to FIGS. 6, 6A, and 6B, the electrochemical oxygen generator/concentrator 11 is illustrated. In the illustrated embodiment, the unique design of the concentrator provides a sturdier pumping unit. For example, there are times when the oxygen delivery tubing may become kinked or occluded, and in prior art concentrators, the concentrator would rupture as a result of any slight backpressure. The concentrator of the embodiments disclosed herein does not rupture when the discharge is occluded. In addition, the system is configured to provide alarms to notify the patient that the oxygen flow/pressure is out of range, thus allowing the patient to check the delivery tubing. If multiple tubings to multiple wound sites are provided, the system may provide alarms to notify the patient which of those tubings includes an oxygen flow/pressure that is out of range, thus allowing the patient to check which of the plurality of delivery tubings triggered the alarm. When a blockage of the oxygen flow occurs, an audible alarm may sound and, in some embodiments, a visual warning light may illuminate. For example, the alarm may sound for two minutes. In an embodiment, the alarm may be muted by pressing a mute button on the monitoring housing, which may operate to mute the audible alarm for fifteen minutes while the user troubleshoots the system, which may include inspecting the tubing for kinks or objects pressing on the tubing, or occlusion that may have occurred at the wound site under the dressing.

FIG. 6 is a top plan view of concentrator 11 showing the cathode plate 70 overlaying the anode plate 72. FIG. 6A is a side elevation view of the concentrator 11. Each of the charged plates may include a carbon backed metalized substrate with an 0.85 sq. inch titanium mesh plated on the woven fiber carbon membrane, which may provide a complete coverage area for electrical conductance to a NAFION^{®} oxygen transfer membrane (NAFION^{®} is a registered trademark of DuPont and is a sulfonated tetrafluroethylene copolymer that is well known in the art as a proton conductor for proton exchange membranes (PEMs)).

FIG. 6B is an exploded perspective view of the concentrator 11. The PEM membrane 74 may be compressed fully between the cathode 70 and the anode 72 by torquing screw type fasteners. An elbow 80 may be attached to the anode plate 72. Electrical contact and transfer to the plates may be accomplished by attaching a copper strip to the titanium mesh substrate. The compressive force applied may provide the necessary adhesion to the surfaces of the two metals. The strips may then be attached to the charge plates with epoxy.

Ambient air may enter the concentrator through inlet 82 which is covered by a filter membrane 84 that allows water vapor and gases to pass through, while preventing contaminants from entering the concentrator. Concentrated O2 may then be discharged out discharge valve 80 which communicates with discharge 54 in housing 13.

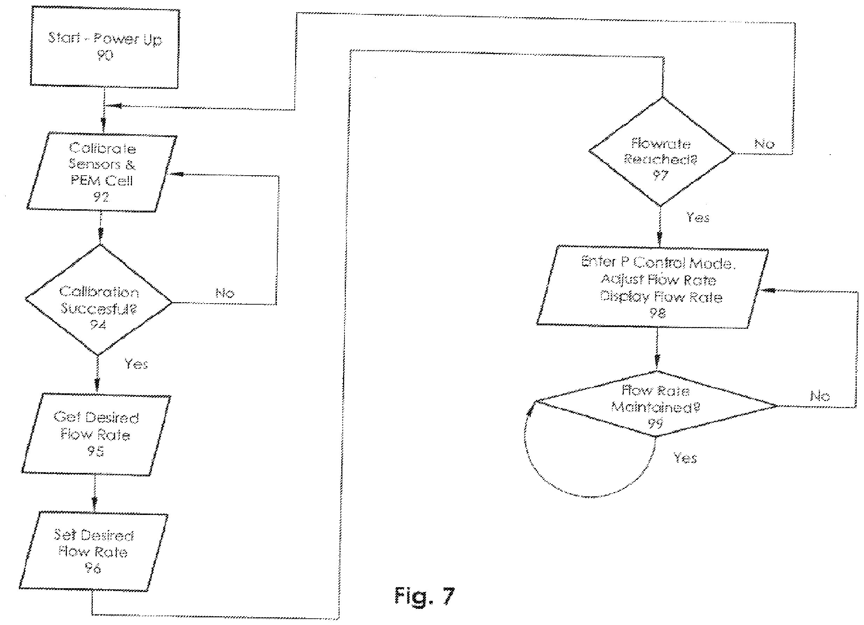

FIG. 7 illustrates a flow chart of a method for controlling tissue oxygenation using the systems discussed above. When the monitoring unit 10 is started or powered up at block 90, the microprocessor 58 may calibrate all sensors and the PEM cell at block 92. Because every PEM cell and each sensor has its own particular functional characteristics, embodiments described herein calibrate the sensors and cell to ensure precise flow rates.

If the calibration is successful at block 94, the microprocessor may then retrieve the desired flow rate at block 95 from the user. In one embodiment, the desired flow rate may be received remotely from a caregiver (e.g., a doctor or nurse of the patient) or a manufacturer of the monitoring unit 10. The microprocessor may then calculate current to output from the PEM to set a desired flow rate at block 96. The microprocessor may then receive input from the flow rate sensor 54 and determines if the set flow rate has been reached at block 97 and, if not, the processor may again seeks to vary current to the sensors and the PEM cell. If the set flow rate is reached at block 97, then the microprocessor may enters a PID control mode at block 98. The flow rate may then be adjusted based upon input from the pressure monitoring system and flow sensors. The microprocessor may also display the flow rate and the temperature (where appropriate) on the monitor display screen 68. In a proportional control mode, the microprocessor may continuously tests the actual flow rate to ensure that it is maintained at block 99 using a feedback loop which looks at variations in sensor and PEM cell efficiencies.

In one embodiment, the flow rate may be adjustable up to a set ceiling, which may provide for a relatively high flow rate. For example, the flow rate may be set to such relatively high levels to treat entire limbs or portions of a patient's body, which may require flow rates in excess of several hundred ml/hr. In such embodiments, a flow rate of 100 ml/hr may be sufficient for a majority of applications, although higher flow rates are envisioned as falling within the scope of the present disclosure. In embodiments where multiple tubing is used to deliver oxygen to multiple wound sites, the flow rate to any particular tubing to particular wound site may be set to be different from that of tubing to other wound site, and may be based on the wound and/or wound characteristics. To achieve higher flow rates, the monitoring unit 10 may be provided with larger batteries or PEM cells to provide for a longer operating time between recharging. For example, the monitoring unit 10 may be provided with sufficient batteries to operate for 24 hours without recharging. In addition, multiple PEM cells or batteries may be placed in parallel to achieve a higher output flow rate where such flow rates are desired (e.g., for larger wounds, multiple wounds, etc.)

In some embodiments, a humidity pack may be provided with the concentrator 11 within the housing 13. In some examples, the humidity pack may be inserted into the monitoring unit 10 when a detected humidity is low (e.g., below a threshold), or may be present in the housing 13 and only activated when a detected humidity is low, or alternatively, may always be active to maintain a desired humidity within the housing 13. In some embodiments, the monitoring unit 10 may monitor the humidity of air to the fuel cell (i.e., the PEM cell), and may warn a user when humidity falls below a level which may potentially impact the function or efficiency of the device. For example, when humidity falls too low, the PEM cell may dry out and become less efficient, which may require additional power to achieve the same flow rate. Thus, if humidity is too low, the flow rate may decrease because there may be a limit as to how much power can be used to compensate for low humidity, and the humidity pack may be utilized to ensure proper humidity in the housing to prevent such occurrences.

In some embodiments, humidity packs may be provided as separate modules and in some instances may be attached to a battery pack. In some embodiments, the humidity pack is disposable and may be discarded after use. In other embodiments, the humidity pack may be rechargeable by adding water to the humidity pack. For example, a rechargeable humidity pack may include a separate recharging chamber from which water may be wicked as needed to recharge the humidity pack. As such, a monitoring or warning system may be included in the device to warn a user when humidity falls below a threshold via audible and/or visual signals, and that warning system may also use graphical, audible, or text instructions to assist a user with procedures to replace or recharge the humidity pack as discussed above, or with any associated troubleshooting as required.

In some embodiments, the battery of the monitoring unit 10 within the housing 13 is replaceable, while in other embodiments, the battery of the monitoring unit 10 within the housing 13 may be rechargeable.

In some embodiments, the processor of the monitoring unit 10 may record and report data to a manufacturer, a caregiver, and or any other entity that is related to the patient, the device, the use of the device, and/or any other information generated as discussed above. For example, the monitoring unit 10 may record and report any or all of: a patient's compliance with offloading, a patient's compliance with changing a wound dressing, compliance with proper connection of the tubing to the dressing, compliance with application of the dressing to the wound, and/or other compliance information known in the art. Furthermore, activity levels of the patient may be recorded. Further still, state information of the monitoring unit 10 such as the time and duration of the monitoring unit 10 being on, whether the device is properly charged, performance of the device (e.g., efficiency, flow output, or other characteristics), environmental conditions (e.g., humidity or temperature), device location (e.g., determined using global positioning satellites (GPS), Bluetooth, Wi-Fi, etc.), and/or other device state information known in the art may be recorded and reported.

In one embodiment, reporting alerts based on the recordation of data may be sent to the patient, a caregiver, a manufacturer of the device, and/or other entities as desired. In some embodiments, the reporting alerts may be sent depending on the severity of the recorded information. For example, for issues that cannot be rectified by the patient, such as device performance warnings, alerts may only be transmitted to the manufacturer of the device, who may be able to intervene if there are indications that a device may have become compromised and needs replacement. Similarly, if the wound monitoring system experiences issues that could affect the patient or the patient's behavior, such as compliance with offloading or dressing changes, alerts may be transmitted to the patient and the caregiver, allowing either the patient or the caregiver (or both) to intervene earlier to ensure optimal outcomes. In some embodiments, congratulatory or encouraging notices or alerts may be sent to the patient if the patient is complying with caregiver or device recommendations, enforcing and ensuring the patient's future compliance. As such, any information recorded and/or reported may be analyzed by the device or another system in order to determine whether alerts or other reporting should be sent to any entities.

In accordance with some embodiments, the wound monitoring system or the monitoring unit may include communication capabilities provided by a communication device 70 such as, for example, a GSM or LTE cellular communication device. Additionally, the wound monitoring system or monitoring unit having a communication device 70 that may include a Wi-Fi (e.g., 802.11 a/b/c/d/n) communication device. The wound monitoring system or monitoring unit may also include near field communication or other radio frequency communication capabilities, such as Bluetooth, to enable close-range data transmission between the wound monitoring system or monitoring unit and other devices, such as a patient's or caregiver's mobile telephone or other computing system.

The wound monitoring system or monitoring unit may include various power control features implemented by the power management system 52. In an embodiment, the monitoring unit may remain powered on once activated, and may require a specific override from a patient to power off the device. For example, the specific override may include a different button for turning off the device than is used to turn the device on. In another example, the monitoring unit may request confirmation from the patient to power the device off. In yet another example, the monitoring unit may require the patient to depress a power off button for a duration of time (e.g., two seconds) before the device is powered off. While a few examples have been provided, a variety of other power control features are envisioned as falling within the scope of the present disclosure.

Other control features may also be included in some embodiments of the wound monitoring system or monitoring unit. In an embodiment, if the system includes a communication device which connects the system to a network, the wound monitoring system may be remotely powered on, powered off, and controlled in a variety of manners. For example, the wound monitoring system may be turned off remotely to enforce payment requirements that allow for the use of the device, e.g., if a patient associated with the device has not provided sufficient payment for the device or ongoing rental of the device. In another example, the wound monitoring system may be turned off remotely if the device is reported as lost or stolen. In some embodiments, the manufacturer of the device or a caregiver may require periodic communication with the wound monitoring system to ensure compliance or authorization for continued use. For example, if too much time has elapsed from a previous communication with the device, the device may be remotely deactivated as a precaution. In another example, the device can also be remotely rebooted or power cycled for troubleshooting by technicians or the manufacturer.

In some embodiments, the wound monitoring system or monitoring unit further includes a relative humidity sensor that may facilitate the monitoring of relative humidity levels which may, in some situations, be used to trigger various other events such as the activation of a humidity pack, the sending of diagnostic data or performance data collected by the device, the requesting of assistance from a caregiver or manufacturer, the provision of warning signals to the patient, caregiver, or manufacturer, and/or other alerts or events known in the art.

In some embodiments, the monitor display screen 68 may provide additional information to the patient or caregiver such as, for example, feedback information. For example, feedback information may include automated warnings from the device such as low battery warnings, blockage warnings, low humidity warnings, etc.; information from the manufacturer such as server-side feedback, or customer service-type feedback such as positive reinforcement for compliance with instructions, warnings of potential issues (e.g., when the tubing or dressing is not properly connected), non-compliance warnings, and/or other feedback information known in the art.

In some embodiments, additional components, devices, or sensors may be used for temperature sensing or detection in addition to the temperature sensors that may be provided within the tubing as discussed above. For example, thermocouples, thermistors, wheatstone bridges, and/or other temperature measuring subsystems may be provided within or coupled to the monitoring unit 10. Such temperature measuring subsystems may be incorporated into the wound dressing, external to the housing, and/or coupled to the patient away from the wound site. For example, separate temperature measuring subsystems may be placed directly into a wound bed, inserted into a wound tunnel, or placed above a dressing material, such as in a non-adherent layer of the dressing. Additionally, for separate temperature measuring subsystems, a notation or other indication may be made in the memory of the device as to the location of the temperature measuring subsystem to permit tracking and reporting of potential temperature offsets from the wound site that may be determined based on the different locations of the temperature measuring subsystems. In some embodiments, the microprocessor or other component of the device may include the capability to detect different types of dressing or temperature measuring subsystems that have been attached to the device.

In some embodiments, the temperature measuring subsystems may be provided in the form of a flat tape, a small bulb, a rod, or other temperature measuring subsystem configurations known in the art. Such temperature measuring subsystems may be used to generate temperature measuring sensor data may be used to monitor wounds for changes in average temperature over time, which in some cases may indicate changes in the healing status of the wound. For example, infection or new capillary growth may be evident from temperature changes. Temperature data may also be used to monitor patient compliance, such as whether the dressing or temperature measuring sensor is placed properly on the wound (or placed on the wound at all), how frequently the dressing is changed, whether the patient is opening the dressing frequently to look at the wound, and/or other compliance related events known in the art. In one embodiment, the data from the temperature measuring sensor may be combined with data from any of the other sensors discussed above (e.g., perfusion data, pH data, pressure data, etc.) to refine analysis as to patient compliance, wound status, and/or other factors.

In some embodiments, additional components, devices, or sensors other than those discussed above may be used to detect pH levels. For example, pH sensors may be provided that are independent from a wound dressing as well as incorporated into the wound dressing. Such pH sensors may be placed directly into a wound bed, inserted into a wound tunnel, or placed above dressing material, such as in a non-adherent layer. In some embodiments, notations or other indication may be made in the memory of the device as to the location of the different pH sensor to permit tracking of potential offsets due to the different locations of the pH sensors. For example, if a pH sensor is incorporated into a wound dressing, the type of dressing and type of pH sensor may be entered into a memory of the device. In some embodiments, the device may include the capability to detect different types of dressings or pH sensor that have been attached to the device.

In some embodiments, the pH sensor may be provided as a flat tape, a small bulb, a rod, or other pH sensor configurations known in the art. The pH sensor data may be used to monitor wounds for changes in pH level over time, which may indicate a change in healing status of the wound. For example, pH changes may indicate new capillary growth or infection. Additionally, data from a pH sensor may be used to monitor patient compliance, such as whether the dressing or pH sensor is placed properly on the wound (or placed on the wound at all.) For example, a pH sensor may require liquid to function, and may provide a different output if the wound is dried out. Data from the pH sensor may also be used to determine the frequency of wound cleansing, and/or may be combined with data from temperature, perfusion, or pressure probes or sensors to further refine determination of patient compliance or wound status analysis.

In some embodiments, additional components, devices, or sensors may be used to monitor perfusion levels. Perfusion monitoring may include methods of detecting the relative and/or absolute amount of blood flowing into a wound, and in some embodiments may further include detecting the relative and/or absolute oxygen saturation of tissue in the wound bed. For example, a perfusion monitoring sensor may include a reflective photoplethysmograph. Similarly to the temperature monitoring sensors and the pH sensors described above, the perfusion monitoring sensor may be independent from a wound dressing as well as incorporated into a wound dressing. For example, a separate perfusion monitoring sensor may be placed directly into a wound bed, inserted into a wound tunnel, or placed above a dressing material, such as in a non-adherent layer. Additionally, a notation or other indication may be made in the memory of the device as to the location of different perfusion monitoring sensors to permit tracking of potential perfusion offsets from the wound site due to the different locations of the perfusion monitoring sensor. In one embodiment, the microprocessor or other component of the device may include the capability to detect different types of dressings or perfusion monitoring sensors that have been attached to the device.

In some embodiments, the perfusion monitoring sensor may have the form of a flat tape, a small bulb, a rod, or other perfusion monitoring sensor configurations known in the art. The perfusion monitoring sensor data may be used to monitor wounds for changes in wound perfusion over time, which in some cases may indicate changes in the healing status of the wound. For example, infection or new capillary growth may be evident from perfusion changes. Perfusion data may also be used to monitor patient compliance, such as whether the dressing or perfusion monitoring sensor is placed properly on the wound (or placed on the wound at all), how frequently the dressing is changed, whether the patient is opening the dressing to look at the wound, and/or other compliance related events. In some embodiments, the data from the perfusion monitoring sensor may be combined with data from other sensors, such as the temperature data, pH data, or pressure data discussed above to further refine determination of patient compliance or wound status analysis.

As such, the wound monitoring system allows patient data and therapy commands to be communicated between the device and a care giver or patient for processing by means of a data input key pad 64 and function control buttons 65. A data port 66 may be used to upload or download data. The monitoring system allows for collection and monitoring of key medical parameters to aid the caregiver in managing the patient care and potentially accelerate the healing process with improved access to more data. Available patient data and device functions are displayed and where appropriate are visually and audibly alarmed on the device function display screen 68. A digital camera 69 may also be utilized to aid the monitoring process visually tracking the wound closure progress.

Although illustrative embodiments have been shown and described, a wide range of modification, change and substitution is contemplated in the foregoing disclosure and in some instances, some features of the embodiments may be employed without a corresponding use of other features. Accordingly, it is appropriate that the appended claims be construed broadly and in a manner consistent with the scope of the embodiments disclosed herein.

## Claims

1. A wound treatment system, comprising:
a housing (13);
a processor (58) located in the housing;
a pressure monitoring system coupled to the processor;
a power delivery system (52) located in the housing and coupled to the processor;
an oxygen concentrator (11) located in the housing and coupled to the power delivery system, wherein the oxygen concentrator includes a plurality of oxygen outlets;
wherein the processor is configured to:
receive pressure information from the pressure monitoring system that is indicative of a pressure in a restricted airflow enclosure provided by a dressing (14, 16) and located adjacent a wound site (20); and
use the pressure information to control the power provided from the power delivery system to the oxygen concentrator in order to control an oxygen flow created by the oxygen concentrator and provided through one of the plurality of oxygen outlets to the restricted airflow enclosure.

2. The system of claim 1, further comprising:
a humidity pack coupled to the housing (13),
a humidity monitoring system (38a) coupled to the processor;
wherein the processor (58) is configured to:
receive humidity information from the humidity monitoring system that is indicative of a humidity in the restricted airflow enclosure of the housing;
use the humidity information to control the humidity provided from the humidity pack to the oxygen concentrator (11); and
use the humidity information to control the power provided from the power delivery system (52) to the oxygen concentrator in order to control an oxygen flow created by the oxygen concentrator and provided through one of the plurality of oxygen outlets to the restricted airflow enclosure.

3. The system of claim 1, further comprising:
a temperature sensor (32a/21a) that is coupled to the processor (58), wherein the processor is configured to receive temperature information from the temperature sensor that is indicative of the temperature of the wound site, and determine whether the oxygen flow from the oxygen concentrator (11) is at a desired oxygen flow level.

4. The system of claim 1, further comprising:
a pH sensor (34a) that is coupled to the processor (58), wherein the processor is configured to receive pH information from the pH sensor that is indicative of the pH of the wound site, and determine whether the oxygen flow from the oxygen concentrator (11) is at a desired oxygen flow level.

5. The system of claim 1, further comprising:
a perfusion sensor (36a) that is coupled to the processor (58), wherein the processor is configured to receive perfusion information from the perfusion sensor that is indicative of the perfusion of the wound site, and determine whether the oxygen flow from the oxygen concentrator (11) is at a desired oxygen flow level.

6. The system of claim 1, wherein the plurality of oxygen outlets includes at least two oxygen outlets, and wherein the processor (58) is configured to:
control an oxygen flow created by the oxygen concentrator (11) and provided through a first oxygen outlet to the restricted airflow enclosure at a first level; and
control an oxygen flow created by the oxygen concentrator and provided through a second oxygen outlet to a second restricted airflow enclosure at a second level different from the first level.

7. The system of claim 1, further comprising:
a battery pack coupled to the housing (13).

8. The system of claim 1, further comprising:
a communications interface, wherein the processor is configured to control the power provided from the power delivery system to the oxygen concentrator in response to an instruction received via the communications interface.

9. The system of claim 1, further comprising:
an alarm system coupled to the pressure monitoring system, wherein the processor is configured to activate the alarm system in response to an out of range indication from the pressure monitoring system.

10. The system of claim 1, further comprising:
a delivery tubing (12) including a first end coupled to the oxygen outlet and a second end that is configured to be positioned in the restricted airflow enclosure adjacent the wound site.

11. A wound treatment system, comprising:
a processor (58);
a plurality of pressure monitoring systems coupled to the processor;
a plurality of oxygen concentrators (11) coupled to the processor; and
a plurality of delivery tubings (12) coupled to the oxygen concentrator and to a plurality of restricted airflow enclosures provided adjacent a plurality of wound sites by a plurality of dressings (14, 16) positioned over each wound site (20);
wherein the processor is configured to:
receive pressure information from the respective pressure monitoring system that is indicative of a pressure in a respective restricted airflow enclosure adjacent a respective wound site; and
use the respective pressure information to control an oxygen flow created by the respective oxygen concentrator and provided through a respective delivery tubing to the respective restricted airflow enclosure.

12. The system of claim 11, further comprising:
a power delivery system (52) coupled to the processor and the respective oxygen concentrator, wherein the processor is configured to use the respective pressure information to control the oxygen flow created by the respective oxygen concentrator (11) by using the respective pressure information to control the power provided from the power delivery system to the respective oxygen concentrator in order to control the oxygen flow created by the respective oxygen concentrator.

13. The system of claim 11, wherein the pressure monitoring system includes a plurality of pressure sensors (19a, 30a), each pressure sensor located at least partially in a respective delivery tubing and that is configured to determine pressure information that is indicative of the pressure in a respective restricted airflow enclosure adjacent a respective wound site.

14. The system of claim 11, wherein the pressure monitoring system includes a pressure sensor that is located in a housing that houses the oxygen concentrator and from which each of the plurality of delivery tubings extend, and wherein the pressure sensor is configured to determine pressure information that is indicative of the pressure in each restricted airflow enclosure adjacent each wound site.

## Patentansprüche

1. Wundbehandlungssystem, das Folgendes umfasst:
ein Gehäuse (13);
einen Prozessor (58), der in dem Gehäuse angeordnet ist;
ein Drucküberwachungssystem, das mit dem Prozessor gekoppelt ist;
ein Stromversorgungssystem (52), das in dem Gehäuse angeordnet ist und mit dem Prozessor gekoppelt ist;
einen Sauerstoffkonzentrator (11), der in dem Gehäuse angeordnet ist und mit dem Stromversorgungssystem gekoppelt ist, wobei der Sauerstoffkonzentrator mehrere Sauerstoffauslässe umfasst;
wobei der Prozessor eingerichtet ist, um:
Druckinformationen aus dem Drucküberwachungssystem zu empfangen, die für einen Druck in einer Umfassung mit eingeschränktem Luftdurchsatz bezeichnend sind, die durch einen Verband (14, 16) bereitgestellt ist und einem Wundort (20) benachbart angeordnet ist; und
die Druckinformationen zu verwenden, um die Leistung zu steuern, die dem Sauerstoffkonzentrator von dem Stromversorgungssystem bereitgestellt wird, um einen Sauerstoffstrom zu steuern, der von dem Sauerstoffkonzentrator erzeugt wird und der Umfassung mit eingeschränktem Luftdurchsatz durch einen der mehreren Sauerstoffauslässe bereitgestellt wird.

2. System nach Anspruch 1, das ferner Folgendes umfasst:
eine Feuchtigkeitspackung, die mit dem Gehäuse (13) gekoppelt ist,
ein Feuchtigkeitsüberwachungssystem (38a), das mit dem Prozessor gekoppelt ist;
wobei der Prozessor (58) eingerichtet ist, um:
Feuchtigkeitsinformationen von dem Feuchtigkeitsüberwachungssystem des Gehäuses zu empfangen, die für eine Feuchtigkeit in der Umfassung mit eingeschränktem Luftdurchsatz bezeichnend sind;
die Feuchtigkeitsinformationen zu verwenden, um die Feuchtigkeit zu steuern, die dem Sauerstoffkonzentrator (11) von der Feuchtigkeitspackung bereitgestellt wird; und
die Feuchtigkeitsinformationen zu verwenden, um die Leistung zu steuern, die dem Sauerstoffkonzentrator von dem Stromversorgungssystem (52) bereitgestellt wird, um einen Sauerstoffstrom zu steuern, der von dem Sauerstoffkonzentrator erzeugt wird und der Umfassung mit eingeschränktem Luftdurchsatz durch einen der mehreren Sauerstoffauslässe bereitgestellt wird.

3. System nach Anspruch 1, das ferner Folgendes umfasst:
einen Temperatursensor (32a/21a), der mit dem Prozessor (58) gekoppelt ist, wobei der Prozessor eingerichtet ist, um Temperaturinformationen von dem Temperatursensor zu empfangen, die für die Temperatur des Wundorts bezeichnend sind, und zu bestimmen, ob der Sauerstoffstrom aus dem Sauerstoffkonzentrator (11) eine erwünschte Sauerstoffstromstärke aufweist.

4. System nach Anspruch 1, das ferner Folgendes umfasst:
einen pH-Sensor (34a), der mit dem Prozessor (58) gekoppelt ist, wobei der Prozessor eingerichtet ist, um pH-Informationen von dem pH-Sensor zu empfangen, die für den pH-Wert des Wundorts bezeichnend sind, und zu bestimmen, ob der Sauerstoffstrom aus dem Sauerstoffkonzentrator (11) eine erwünschte Sauerstoffstromstärke aufweist.

5. System nach Anspruch 1, das ferner Folgendes umfasst:
einen Perfusionssensor (36a), der mit dem Prozessor (58) gekoppelt ist, wobei der Prozessor eingerichtet ist, um Perfusionsinformationen von dem Perfusionssensor zu empfangen, die für die Perfusion des Wundorts bezeichnend sind, und zu bestimmen, ob der Sauerstoffstrom aus dem Sauerstoffkonzentrator (11) eine erwünschte Sauerstoffstromstärke aufweist.

6. System nach Anspruch 1, wobei die mehreren Sauerstoffauslässe mindestens zwei Sauerstoffauslässe umfassen und wobei der Prozessor (58) eingerichtet ist, um:
einen Sauerstoffstrom zu steuern, der von dem Sauerstoffkonzentrator (11) erzeugt wird und der Umfassung mit eingeschränktem Luftdurchsatz durch einen ersten Sauerstoffauslass in einer ersten Stärke bereitgestellt wird; und
einen Sauerstoffstrom zu steuern, der von dem Sauerstoffkonzentrator erzeugt wird und einer zweiten Umfassung mit eingeschränktem Luftdurchsatz durch einen zweiten Sauerstoffauslass in einer zweiten Stärke bereitgestellt wird, die von der ersten Stärke verschieden ist.

7. System nach Anspruch 1, das ferner Folgendes umfasst:
einen Batteriesatz, der mit dem Gehäuse (13) gekoppelt ist.

8. System nach Anspruch 1, das ferner Folgendes umfasst:
eine Kommunikationsschnittstelle, wobei der Prozessor eingerichtet ist, um die Leistung, die dem Sauerstoffkonzentrator von dem Stromversorgungssystem bereitgestellt wird, als Reaktion auf einen Befehl zu steuern, der über die Kommunikationsschnittstelle empfangen wurde.

9. System nach Anspruch 1, das ferner Folgendes umfasst:
ein Alarmsystem, das mit dem Drucküberwachungssystem gekoppelt ist, wobei der Prozessor eingerichtet ist, um das Alarmsystem als Reaktion auf einen nicht bereichskonformen Anzeigewert aus dem Drucküberwachungssystem zu aktivieren.

10. System nach Anspruch 1, das ferner Folgendes umfasst:
einen Versorgungsschlauch (12), der ein erstes Ende, das mit dem Sauerstoffauslass gekoppelt ist, und ein zweites Ende umfasst, das eingerichtet ist, um in der Umfassung mit eingeschränktem Luftdurchsatz dem Wundort benachbart positioniert zu sein.

11. Wundbehandlungssystem, das Folgendes umfasst:
einen Prozessor (58);
mehrere Drucküberwachungssysteme, die mit dem Prozessor gekoppelt sind;
mehrere Sauerstoffkonzentratoren (11), die mit dem Prozessor gekoppelt sind; und
mehrere Versorgungsschläuche (12), die mit dem Sauerstoffkonzentrator und mit mehreren Umfassungen mit eingeschränktem Luftdurchsatz gekoppelt sind, die durch mehrere Verbände (14, 16), die über jedem Wundort (20) positioniert sind, mehreren Wundorten benachbart bereitgestellt sind;
wobei der Prozessor eingerichtet ist, um:
Druckinformationen aus dem jeweiligen Drucküberwachungssystem zu empfangen, die für einen Druck in einer jeweiligen, einem jeweiligen Wundort benachbarten Umfassung mit eingeschränktem Luftdurchsatz bezeichnend sind; und
die jeweiligen Druckinformationen zu verwenden, um einen Sauerstoffstrom zu steuern, der von dem jeweiligen Sauerstoffkonzentrator erzeugt wird und der jeweiligen Umfassung mit eingeschränktem Luftdurchsatz durch einen jeweiligen Versorgungsschlauch bereitgestellt wird.

12. System nach Anspruch 11, das ferner Folgendes umfasst:
ein Stromversorgungssystem (52), das mit dem Prozessor und dem jeweiligen Sauerstoffkonzentrator gekoppelt ist, wobei der Prozessor eingerichtet ist, um die jeweiligen Druckinformationen zu verwenden, um den Sauerstoffstrom zu steuern, der von dem jeweiligen Sauerstoffkonzentrator (11) erzeugt wird, indem die jeweiligen Druckinformationen verwendet werden, um die Leistung zu steuern, die dem jeweiligen Sauerstoffkonzentrator von dem Stromversorgungssystem bereitgestellt wird, um den Sauerstoffstrom zu steuern, der von dem jeweiligen Sauerstoffkonzentrator erzeugt wird.

13. System nach Anspruch 11, wobei das Drucküberwachungssystem mehrere Drucksensoren (19a, 30a) umfasst, jeder Drucksensor mindestens teilweise in einem jeweiligen Versorgungsschlauch angeordnet ist und eingerichtet ist, um Druckinformationen zu bestimmen, die für den Druck in einer jeweiligen, einem jeweiligen Wundort benachbarten Umfassung mit eingeschränktem Luftdurchsatz bezeichnend sind.

14. System nach Anspruch 11, wobei das Drucküberwachungssystem einen Drucksensor umfasst, der in einem Gehäuse angeordnet ist, das den Sauerstoffkonzentrator beherbergt und aus dem sich jeder der mehreren Versorgungsschläuche erstreckt, und wobei der Drucksensor eingerichtet ist, um Druckinformationen zu bestimmen, die für den Druck in jeder, jedem Wundort benachbarten Umfassung mit eingeschränktem Luftdurchsatz bezeichnend sind.

## Revendications

1. Système de traitement de plaie comprenant :
un boîtier (13) ;
un processeur (58) situé dans le boîtier ;
un système de surveillance de la pression couplé au processeur ;
un système de distribution de puissance (52) situé dans le boîtier et couplé au processeur ;
un concentrateur d'oxygène (11) situé dans le boîtier et couplé au système de distribution de puissance, le concentrateur d'oxygène comprenant une pluralité de sorties d'oxygène ;
le processeur étant configuré pour :
recevoir en provenance du système de surveillance de la pression des informations de pression qui indiquent une pression dans une enceinte à flux d'air restreint fournie par un pansement (14, 16) et située de manière adjacente à un site de plaie (20) ; et
utiliser les informations de pression pour commander la puissance fournie par le système de distribution de puissance au concentrateur d'oxygène afin de commander un flux d'oxygène créé par le concentrateur d'oxygène et fourni par l'une de la pluralité de sorties d'oxygène à l'enceinte à flux d'air restreint.

2. Système selon la revendication 1, comprenant en outre :
un pack d'humidité couplé au boîtier (13),
un système de surveillance de l'humidité (38a) couplé au processeur ;
le processeur (58) étant configuré pour :
recevoir en provenance du système de surveillance de l'humidité des informations sur l'humidité qui sont indicatives de l'humidité dans l'enceinte à flux d'air restreint du boîtier ;
utiliser les informations sur l'humidité pour commander l'humidité fournie au concentrateur d'oxygène (11) par le pack d'humidité ; et
utiliser les informations sur l'humidité pour commander la puissance fournie par le système de distribution de puissance (52) au concentrateur d'oxygène afin de commander un flux d'oxygène créé par le concentrateur d'oxygène et fourni par l'une de la pluralité de sorties d'oxygène à l'enceinte à flux d'air restreint.

3. Système selon la revendication 1, comprenant en outre :
un capteur de température (32a/21a) qui est couplé au processeur (58), le processeur étant configuré pour recevoir des informations de température en provenance du capteur de température qui sont indicatives de la température du site de plaie, et déterminer si le flux d'oxygène du concentrateur d'oxygène (11) est à un niveau de flux d'oxygène souhaité.

4. Système selon la revendication 1, comprenant en outre :
un capteur de pH (34a) qui est couplé au processeur (58), le processeur étant configuré pour recevoir des informations de pH du capteur de pH qui sont indicatives du pH du site de plaie, et déterminer si le flux d'oxygène du concentrateur d'oxygène (11) est à un niveau de flux d'oxygène souhaité.

5. Système selon la revendication 1, comprenant en outre :
un capteur de perfusion (36a) qui est couplé au processeur (58), le processeur étant configuré pour recevoir des informations de perfusion en provenance du capteur de perfusion qui sont indicatives de la perfusion du site de plaie, et déterminer si le flux d'oxygène en provenance du concentrateur d'oxygène (11) est à un niveau de flux d'oxygène souhaité.

6. Système selon la revendication 1, la pluralité de sorties d'oxygène comprenant au moins deux sorties d'oxygène, et le processeur (58) étant configuré pour :
commander un flux d'oxygène créé par le concentrateur d'oxygène (11) et fourni par une première sortie d'oxygène à l'enceinte à flux d'air restreint à un premier niveau ; et
commander un flux d'oxygène créé par le concentrateur d'oxygène et fourni par une seconde sortie d'oxygène à une seconde enceinte à flux d'air restreint à un second niveau différent du premier niveau.

7. Système selon la revendication 1, comprenant en outre :
une batterie couplée au boîtier (13).

8. Système selon la revendication 1, comprenant en outre :
une interface de communication, le processeur étant configuré pour commander la puissance fournie par le système de distribution de puissance au concentrateur d'oxygène en réponse à une instruction reçue par l'intermédiaire de l'interface de communication.

9. Système selon la revendication 1, comprenant en outre :
un système d'alarme couplé au système de surveillance de la pression, le processeur étant configuré pour activer le système d'alarme en réponse à une indication hors plage provenant du système de surveillance de la pression.

10. Système selon la revendication 1, comprenant en outre :
une tubulure d'administration (12) comprenant une première extrémité couplée à la sortie d'oxygène et une seconde extrémité configurée pour être positionnée dans l'enceinte à flux d'air restreint de manière adjacente au site de plaie.

11. Système de traitement de plaie comprenant :
un processeur (58) ;
une pluralité de systèmes de surveillance de la pression couplés au processeur ;
une pluralité de concentrateurs d'oxygène (11) couplés au processeur ; et
une pluralité de tubulures d'administration (12) couplées au concentrateur d'oxygène et à une pluralité d'enceintes à flux d'air restreint fournies de manière adjacente à une pluralité de sites de plaie par une pluralité de pansements (14, 16) positionnés sur chaque site de plaie (20) ;
le processeur étant configuré pour :
recevoir des informations de pression en provenance du système de surveillance de la pression respectif qui sont indicatives d'une pression dans une enceinte à flux d'air restreint respective adjacente à un site de plaie respectif ; et
utiliser les informations de pression respectives pour commander un flux d'oxygène créé par le concentrateur d'oxygène respectif et fourni par une tubulure de distribution de puissance respective à l'enceinte à flux d'air restreint respective.

12. Système selon la revendication 11, comprenant en outre :
un système de distribution de puissance (52) couplé au processeur et au concentrateur d'oxygène respectif, le processeur étant configuré pour utiliser les informations de pression respectives pour commander le flux d'oxygène créé par le concentrateur d'oxygène respectif (11) en utilisant les informations de pression respectives pour commander la puissance fournie par le système de distribution de puissance au concentrateur d'oxygène respectif afin de commander le flux d'oxygène créé par le concentrateur d'oxygène respectif.

13. Système selon la revendication 11, le système de surveillance de la pression comprenant une pluralité de capteurs de pression (19a, 30a), chaque capteur de pression étant situé au moins partiellement dans une tubulure de distribution de puissance respective et étant configuré pour déterminer des informations de pression qui sont indicatives de la pression dans une enceinte à flux d'air restreint respective adjacente à un site de plaie respectif.

14. Système selon la revendication 11, le système de surveillance de la pression comprenant un capteur de pression situé dans un boîtier qui abrite le concentrateur d'oxygène et à partir duquel s'étendent chacun de la pluralité de tubes d'administration, et le capteur de pression étant configuré pour déterminer des informations de pression qui sont indicatives de la pression dans chaque enceinte à flux d'air restreint adjacente à chaque site de plaie.
